# EUROPEAN PATENT SPECIFICATION

(11) **EP 1 790 234 B1**
(45) Date of publication and mention of the grant of the patent: **02.07.2014**
(21) Application number: 06388064.5
(22) Date of filing: 24.11.2006
(51) Int. Cl.: A23K 1/14, A23K 1/16, A23K 1/18, A61P 1/00

(54) **Natural antioxidant additive for feed and drinking water**
Natürlicher Antioxidationzusatz für Futtermittel oder für Trinkwasser
Additif antioxydant naturel pour la nourriture ou pour l'eau potable pour animaux

(30) Priority: 28.11.2005 DK 200501670
(43) Date of publication of application: 30.05.2007
(73) Proprietor: Chr. Hansen A/S, 2970 Hørsholm (DK)
(72) Inventor: Elgaard, Troels, 2660 Brondby Strand (DK); Nielsen, Beatrice Konstanze Klinzing, 2650 Hvidovre (DK)
(74) Representative: Schouboe, Anne

(56) References cited:
- WO-A-99/48386
- WO-A-03/071883
- WO-A-2004/091307
- WO-A-2006/061021
- WO-A1-00/66078
- WO-A1-2005/084625
- WO-A1-2006/016357
- WO-A1-2006/061021
- WO-A1-2007/039262
- WO-A2-2007/149287
- ES-A1- 2 156 718
- FR-A1- 2 772 235
- FR-A1- 2 792 831
- US-A1- 2002 098 253
- DATABASE CABA [Online] 1995, SOOUD, A. O. ET AL: "The possibility of replacing the by-products formulated mixture instead of the conventional diets in feeding camels compared to sheep and goats" XP002422178 retrieved from STN Database accession no. 95:55504 & ANNALS OF AGRICULTURAL SCIENCE (CAIRO), NO. 1 SPECIAL ISSUE, PP. 69-83. 19 REF. ISSN: 0570-1783, 1993,
- CARLUCCIO, M.A., ET AL.: "Olive oil and red wine antooxidant polyphenols inhibit endothelial activation" ARTERIOSCLEROSIS, THROMBOSIS, AND VASCULAR BIOLOGY., vol. 23, 2003, pages 622-629, XP002421919

## Description

### BACKGROUND OF THE INVENTION

The present invention is defined by the claims and concerns both an intestine-functional feed additive with antioxidative properties based on natural or nature identical active substances, and a feed mix that contains the additive, as well as a method for production of domestic animals.

The use of antibiotic or chemotherapeutic substances, referred to as 'growth promoters', in the production of livestock is well known. In recent years attention has increasingly focused on problems related to the widespread use of antibiotics or chemotherapeutics as growth promoters. More and more pathogenic micro-organisms are able to develop resistance to the antibiotics which thus become less effective. There is a risk that residues of antibiotics are left in animal products consumed by humans, whereby microorganisms that cause disease can become resistant. Also, there is a risk of consuming antibiotic-resistant micro-organisms through animal products. Antibiotics may be rediscovered in the ground water and in streams. Finally there is a risk that humans, who produce and use feed containing antibiotics, will have antibiotics-resistant bacteria in their bodies.

In consequence of the above a continued use of antibiotics as growth promoters is, among others, expected to increase the risk of epidemic outbreaks among animals and/or humans, which cannot be treated by antibiotics. A considerable number of humans have already developed allergy towards certain antibiotics. Furthermore, one of the so far used chemo-therapeutic growth promoters has shown to be causing cancer.

The result of this has already been a complete stop of the use of classical growth promoters in swine production in various countries, including Denmark and Sweden, and will be banned within the EU by the end of 2005.

As a consequence of the relations mentioned above, and to ensure the economy and sustainability in livestock farming, new alternative methods are sought to ensure the live-stock's growth conditions free of both disease and use of antibiotics.

Such drugs can advantageously be based on natural or nature identical active substances. To ensure the acceptance of these new additives by the farmer, as well as the feed industry, they should also have the same or even better effect on the animals' growth and feed utilization as the classical growth promoters.

It is well know that many plants contain various functional and/or antibacterial substances, such as saponins, flavonoids, tannins, hydroxycymens and terpenes, and it is also known to exploit such active substances from plant materials in medicine. A considerable part of the positive characteristics of these active substances are caused by their antioxidative characteristics. Antioxidants protect the cells against degradation and harmful substances, reduce the risk of infection, and have a special effect in intensive production systems and stress situations.

Similarly it has been suggested to use such natural substances as daily supplements for both animals and humans. The claimed effects of such active plant components are, how-ever, in many cases not adequately well-documented, and many of the components appear only in small amounts in the concerned plants, just as they are mostly too costly and/or too difficult to access for a preventive daily use as feed additive.

It is well known that olives have a number of health promoting effects, such as lower risk of developing cardiovascular diseases and cancer, which partly has been dedicated the high content of antioxidants of the olives. Among the isolated antioxidants from olive oil are phenol components like simple phenols (hydroxytyrosol ((3.4-dihydroxyphenyl)ethanol), tyrosol (p-hydroxyphenylethanol)), secoiridoids (oleuropein) and lignans (pinoresinol) and other polyphenols ( Owen et al., 2000: Olive-oil consumption and health: the possible role of antioxidants. The Lancet Oncology 1, 107-112). These active substances have shown biological activity, such as inhibition of cell growth of cancer cells and antiviral and antioxidative activity (eg. inhibition of oxidation of low-density lipoproteins (LDL) and inhibition of oxidative stress). The antioxidative activity from the active substances found in olive is far more effective than a synthetic form of vitamin E (Owen et al., 2000: Olive-oil consumption and health: the possible role of antioxidants. The Lancet Oncology 1, 107-112). There has been found in vitro anti-microbial activity in olive leaves (Markin et al. 2003. In vitro anti-microbial activity of olive leaves. Mycoses 46 (3-4): 132-136) and antifungal activity (Del Rio et al. 2003: Enhancement of phenolic compounds in olive plants and their influence on resistance against Phytophthora sp. Food Chemistry 83 (1): 75-78).

It is equally well known that wine and byproducts from wine production have a series of health promoting effects. Wine contains phenols including tannins and some pigments, and also organic acids and vitamins. Chromatographic studies have shown a series of flavan-3-ols and flavanols. Known health promoting effects from wine are antibacterial, antiviral, antialler-gic, antioxidative and cancer-inhibiting (Bombardelli & Morazzoni, 1995: Vitis Vinifera L. Fitoterapia, LXVI (4)).

Various patents describe methods for extracting antioxidants from olive based material (US 2002/0004077 A1 (Cuomo et al)). WO 03/086442 discloses naturally occurring plant compounds, extracts and derivatives, among these oleuropein, for protection of health promoting products for humans. Use of antioxidative substances derived from plant material from olive as a feed additive is proposed in DK utility model application DK BA 2004 00319. Patents with wine encompass a feed additive made solely of a wine by-product (EP 1 419 701 A1) or combined with other mentioned botanical elements containing tannins or flavonoids. Other patents include wine components as feed for animals.

The prior art does not describe a combination of wine and olives according to claim 1 as additives for animal feed.

WO 2006/061021 relates to the use of olive leaf extract as an anti-oxidant in food sources. However, there is no mentioning of any combination product of olive leaf extract with e.g. grape products.

WO 2004/091307 relates to bioactive feed additives with anti-bacterial properties, wherein the additives may be extracts from olive leaf or grape seed extracts. There is, however, no mentioning of the use according to present invention and its anti-oxidative effect and improved feed conversion.

ES 2156718 relates to compositions comprising olive oil and grape extracts for improvement of meat quality in livestock. However, there is no mentioning of any olive leaf extracts or the effect of presently claimed use

WO 2003/071883 relates to probiotic compositions comprising a lactobacillus or bifidobacterium species and which may further comprise an anti-oxidant composition comprising olive oil or red grapes extracts. However, there is no mentioning or relation to the use of present invention and the effects thereof.

Soud et al. (Ann. Agric. Sci., 1993, No. 1, pp. 69-83) relates to animal feeds comprising olive oil pulp and grape pulp for the purpose of improved digestability. However, there is no mentioning of any olive leaf extracts or the effect of presently claimed use.

WO 1999/48386 relates to compositions comprising olive oil and red wine extracts as a support material in food compositions. The document is, however, silent on the use of olive leaf extracts.

FR 2772235 relates to compositions comprising olive oil extracts and grape extracts for use in nutraceutical treatment of cardio vascular diseases. The document is, however, silent on the use of olive leaf extracts and its effect as anti-oxidants and improved feed conversion in animals.

FR 2792831 relates to use of lycopene in an anti-aging composition, wherein extracts of olive and red wine are used to solubilize the lycopene. The document is, however, silent with respect to any use according to present invention resulting in an improved feed conversion in animals.

US 2002/098253 relates to compositions comprising olive leaf extracts, grape seed extracts and extracts of lotus for cosmetic use. The document is however silent with respect to any use according to present invention resulting in an improved feed conversion in animals.

Additionally, many of the mentioned applications and similar applications that include constituents of olives or wine as antioxidant for products for feed for domestic animals, are based on costly extracts. Therefore a cost-effective additive with antioxidative and thus health promoting properties for products for domestic animals is needed. Such product should be more cost- effective than for example a pure olive product, a pure rosemary product or a pure wine-based product.

It has now been found that a product based on a combination of an olive based material and a wine based material is an excellent solution for this problem. The present invention is based on this finding.

### BRIEF DESCRIPTION OF THE INVENTION

In one aspect, the present invention is directed to a natural intestine-functional additive with antioxidative properties for use as an additive in animal feed, and containing natural and/or nature identical active substances including a) a first active component containing active antioxidative substances, and which is derived from olive plant material, and b) a second active component containing antioxidative substances, and that is derived from grape plant material, along with optional conventional carriers and/or additives.

Consequently, present invention relates to use of a natural intestine-functional additive with antioxidative properties containing natural and/or nature-identical active substances comprising;
- a first component comprising one or more of ground leaves of olives (*Olea europaea* L), an extract thereof obtainable by extraction with water, alcohol or a combination thereof, and a residual ground olive leaf product remaining after removal of one or more substances by said extraction, and
- a second component comprising at least one of a primary pomace based on a by-product from wine production, which is a first residual product obtainable by pressing the juice out of the grapes, a secondary pomace, which is a second residual product obtainable by pressing of a residual product after fermentation of the primary pomace, and a tertiary pomace which is a third residual product obtainable by removal of one or more substances by extraction from the secondary pomace, said second component obtainable from grape plants (*Vitis vinifera* L), as an additive for animal feed for improved feed conversion in the animals.

In another aspect, an additive is provided that improves weight gain and feed conversion in production animals and has positive effects on the health of both production animals and pets.

In another aspect, a feed mix is provided for domestic animals containing the additive combined with a conventional feed, and instructions are provided for production of domestic animals when the animals are fed the feed mix.

It is believed that the unexpected result of the inventive combination of materials derived from grape plants and olive plants is at least partially due to a synergistic effect.

A combination of grape plant derivatives and olive plant derivatives can have especially valuable properties since the polyphenols found in olive plants that have anti-oxidative properties are partially soluble in the oil phase and partially soluble in the water phase Hydroxytyrosol is soluble in both the lipid phase and the water phase. The antioxidants in grape plant derivatives are mainly soluble in the water phase. The combination of products from grape plant derivatives and olive plant derivatives results in a product which contains both fat-soluble and water-soluble antioxidants, and also antioxidants that are soluble in both phases, and thereby can act as conveyers between the two phases. A broad-spectred solubility results in increased activity and leads to an increased anti-oxidative effect because the absorbtion of the antioxidant mix is increased. It is believed that further mechanisms which are not yet understood contributes to the surprising results of the inventive combination of olives and wine.

The extent of applicability of the invention appears from the following detailed description.

### DETAILED DESCRIPTION OF THE INVENTION

According to one embodiment of the invention the additive is obtainable from a by-product from the production of olive oil or on basis of olive leaves. When producing olive oil, a by-product referred to as "pulp", and waste water emerge. These products have a high content of antioxidants, which is also the case with leaves from the olive tree that are not exploited on a large scale.

Olive is the fruit of the olive tree (*Olea europaea* L). The production of olive oil is carried out by washing the olive fruits, after which they are crushed in a hammer mill. The obtained "pomace", a mixture of stone and olive pericarp (the flesh itself), is homogenized and moved to platens, where the oil is squeezed out. The water is then separated from the clear oil. More oil fractions can be derived by squeezing the "pomace" repeatedly.

The other active component in the additive is based on a by-product from wine production, which can be the pomace - i.e. anything remaining but the grape juice. As used herein, this pomace is referred to as "the primary pomace".

The primary pomace (also called Grape Pomace) from the wine production has traditionally been used as raw material for production of various products. After each step in the process it is possible to achieve a pressed product that to a certain extent can be used in the additive in the present invention.

In red wine production the grape juice ferments with skin and seeds. After 3-7 days the juice is pressed out and this primary pomace is one possible raw material for the active component.

The primary pomace can then be further processed. For example, the seeds can be separated from the skin with the purpose of oil extraction from the seeds. Both the residual products from the oil extraction, the discarded skin, and the discarded seeds are well suited as raw material for the active component in the present invention.

In certain wine districts there is a tradition for further processing of the skin fraction for extraction of natural colorants and the likes. In connection with most extraction processes the residual products have an increased content of the active substances that are of interest in this invention. It is also possible to further process the grape seeds, the grape skin, or the total pomace to extract the antioxidants.

A preferred residual product of the pressing is based on red grapes. Examples thereof can be of the sorts Cabernet Sauvignon, Merlot, Syrah, Pinot Noir, Lemberger or Tempranillo.

The utilized residual product from the pressing can with advantage be from red grapes used for production of white wine as the remaining grape material is removed from the grape juice as shortly after the pressing as practically possible. Consequently, all the active substances relevant to the natural additive are retained in the residual product of the pressing.

It is common to use the primary residual product of the pressing process for extraction of different fermentation products, such as alcohol, citric acid, tartaric acid, among others. Before fermentation, a fraction of the grape seeds are usually sorted out. The fermentation process produces a residual product, the secondary residual product from the pressing that can be used as the additive or part of the additive in connection with this invention. In the secondary residual product the high content of easily transformed energy has been converted in the fermentation process, while the concentration of the active substances relevant to this invention has been increased.

Further, the concentration of the active substances relevant to this invention is further increased as a result of some conventional extraction processes for the preparation of products such as grape seed oil and natural colorants as enocyanin subjected on the secondary residual product of the pressing. The residue after such extraction process has a specially high concentration of the relevant active substances. As used herein this residue is referred to as "the tertiary residual product from the pressing".

The residual products from the wine production and the waste water from the olive oil production can immediately after the pressing of the juice be dried out to a water content of maximum 8-15 weight%, usually to a maximum water content of 10% water, or optionally 8% water. The products can also be preserved by way of addition of 0 to 10% weight NaCl or another salt, and thereafter it can be sent to drying or other preservation. To secure the stability even further 0-5% of an acid appropriate for feed can be used. Examples of such an acid are citric acid, tartaric acid, lactic acid or a mix of these. The addition of one or more of these acids also has a positive effect on the activity of the product, among others because anthocyanines and citric- and tartaric acid function in synergy. Additionally, it has been found that the addition of sodium benzoate and potassium benzoate is advantageous.

To the extent deemed necessary, the waste water, the residual products from the pressing and the dried and ground olive leaves can be concentrated further by removing some of the water remaining in the products. This further concentration can be done by filtration, extraction, centrifugation or other conventional methods.

Extracts can be produced by extraction of the relevant plant material with water, alcohol extracts or a combination thereof, preferably with water or a water/alcohol mixture.

The extracts can be used in the existing liquid form, or in a powder form manufactured by drying, such as spray drying and/or by absorption and/or by adsorption on an expedient carrier agent.

In principle the plant material can be derived from any of the relevant parts of the olive and/or grape plants, especially the leaves and/or fruits, provided such plant material contains antioxidative substances in a sufficient amount. Examples are olive leaves, by products from olive oil production and by-products from wine production.

Thus, one embodiment of the invention includes a first active component obtained from olives and a second active component obtained from grapes. The olive component is obtained from at least one of following raw materials: dried and ground olive leaves or an extract thereof, the residual product from the pressing of olive oil, a concentrated liquid, any extract derived from the waste water of the olive oil production, and any other products obtained from olive plants. The grape component is obtained from at least one of three raw materials based on the residual products from the pressing, with a higher or lower content of active substances. The grape component can also be obtained from any product that arises out of wine, e.g. an extract product from pomace based raw materials.

In the exemplary embodiment the first and the second components of the additive are each at least one of a liquid, a spray dried powder, and a ground product.

The weight ratio of the olive component (component a) to the wine component (component b) in the inventive additive based on the weight of dried extracts is, in one embodiment, in the range of 99:1 to 1:99, more specifically, 80:20 to 20:80, more specifically still 70:30 to 30:70. In a more specific embodiment, the weight ratio of component a to component b in the inventive additive is 60:40 to 40:60, and more specifically, about 50:50.

As stated the above weight ratios refer to dried extracts. In case the plant material is used in another form, for example if the component is in the form of ground leaves, the amount of that component should be sufficient to provide a similar amount of antioxidative substances as though the component was a dried extract.

In addition, the invention covers all combinations of grape and olive parts, regardless of the technical form or concentration.

Olive leaves and by-products from the production of olive oil together with residual products from the wine pomace contain a series of active substances. Some of these substances are polyphenols having antioxidative properties together with the subgroups anthocyanins and oligomeric procyanidins.

In some embodiments the inventive additive includes fat soluble antioxidative active substances and water soluble antioxidative active substances, and optionally further fat soluble active substances and/or water soluble active substances.

The additive in this invention has at least the following beneficial effects:
1) Used as a feed additive, an antioxidative effect in the animal by protecting against degradation of cells, reducing the usage of other antioxidants, e.g. Vitamin E and Selenium, and protection against heat stress.
2) Improvement of productivity and health status in animals by having antimicrobial and antiviral properties, improving feed conversion, improving the utilization of nutrients, improving health status by protecting the epithelial cells in the gastrointestinal tract, stimulate the animals' immune system as well as improve immune response to vaccination and thereby protect against stress related diseases or a production decline, strengthen especially the smallest and weakest animals and achieve a more homogeneous production.
3) Improvement of product quality by improvement of the water-holding properties (reduction of drip loss) by protecting the cell membranes, improvement of the meat color by delaying the pigment, oxidation from the red oxymyoglobin to the brown metmyoglobin, improvement of the stability of the fatty acids, and thereby the shelf life of the meat, by delaying lipid oxidation through the antioxidative activity of the product, and improvement of meat quality by lowering the risk of developing PSE (pale, soft and exudative) by stress reduction.

The inventive additive can, with great benefit, be used for monogastric animals, ruminants, fish, crustaceans, and pets.

In one embodiment, depending on the concentration and the variation of the active substances in the raw material relevant to the invention, the raw material is mixed with an appropriate carrier. Accordingly, the final product is standardized and diluted such that the compounders and home mixers can mix directly in the feed.

In one embodiment, suitable carrier substances include mineral carrier substances such as clay minerals and zeolites - sepiolit and klinoptilolit. In addition, other suitable substances include vegetable carrier substances such as wheat flour, wheat bran, and soy shell flour as well as other related compounds. Calcium carbonate can also be used as a carrier substance. Another useful carrier substance includes grape seed flour, which is a known carrier substance used frequently in animals' feed.

A combination of the active elements with clay minerals also result in an optimal utilization of the active elements, because of the slow release effect of clay minerals. A combination with clay minerals thus ensures that the active elements are released in the lower part of the intestine.

In other formulations the product includes a combination of liquid from olives and from grapes, as well as a combination of spray dried powders based on the two sources of raw material, without the addition of carrying agents. In one embodiment, liquids as well as spray dried products are given to the animals via drinking water, possibly with a natural emulsifier. In another embodiment, the liquids and/or spray dried products are mixed directly in the feed, for example as a premix.

In the exemplary embodiment, the product contains up to 5 weight% of a feed acceptable organic acid relative to the amount of olive products, by-products and wine by-products measured as dry matter. In addition, the product contains up to 10 weight% of a feed acceptable salt relative to the amount of olive products, by-products and wine by-products measured as dry matter.

In one embodiment, olive leaves, the pomace from olive oil production and the grape pomace are ground before being mixed with the actual carrier substance.

The inventive additive is believed to have a considerable effect on the livestock's health, production efficiency, feed intake, daily weight gain and feed utilization. It is further believed that the inventive additive reduces the usage of other antioxidants such as vitamin E and selenium in production animals and pets, has antimicrobial and antiviral characteristics, improves nitrogen utilization, and improves the nutrient substance utilization generally.

The inventive additive may be used by animal production including animal breeding and production of animal products such as meat, eggs and milk. In one embodiment, the additive is added to the conventional animal feed. Typically the quantity of the additive for 1000 kg feed contains 1-1000 g of the first component and 1-1000 g of the second component, both measured as dry matter, for example as obtained by drying an extract. In a more particular embodiment, the amount of the first component is 1.5 - 500 g, and more particularly still, 2 - 100 g for 1000 kg animal feed. In addition, the amount of the second component is more particularly 1.5 - 500 g, and more particularly still 2 - 100 g for 1000 kg animal feed.

The above weight amounts refer to dried extracts. In case the plant material is used in another form, for example as ground leaves, the amount of the component in question should be one giving a similar amount of the antioxidative substances.

### Examples

To verify the effect of the additive a series of trials have been carried out on selected Danish farms. The present examples use a spray dried aqueous extract from the press residue product obtained immediately after the pressing out of grape juice, in the following named "Wine extract".
- "Nor-Guard OV Powder": Consists of 0.3% Wine extract, 15% dried, grinded olive leaves, 40.5 % sepiolite, and 44.2 % flour
- "Nor-Guard OV 11 Powder": 50% powder of dried extract (ethanol/water, 60:40 v/v) of olive leaves and 50% Wine extract
- "Nor-Guard OV 12 Powder": 33% powder of dried extract (ethanol/water, 60:40 v/v) of olive leaves and 67% Wine extract
- "Nor-Guard OV 21 Powder": 67% powder of dried extract (ethanol/water, 60:40 v/v) of olive leaves and 33 % Wine extract
   "Nor-Guard O Powder": 100% Wine extract
   "Nor-Guard V Powder": 100% powder of dried extract (ethanol/water, 60:40 v/v) of olive leaves

### Example 1 - Nor-Guard OV Powder

Trial on a pig farm on Funen. Trial start at weaning on June 9, 2005. The duration of the trial was one week. A group of medium sized pigs were selected for the trial group. Nor-Guard OV Powder was added to the feed at a dosage of 1000 ppm. The addition of 1000 ppm ZnO that, in this herd, is ordinarily administered in the post weaning period to reduce diarrhea was reduced to 500 ppm in the trial group. The control group was administered 1000 ppm ZnO. Except the administration of ZnO and Nor-Guard OV Powder the two groups were managed in the same manner. Diarrhea at weaning is normally observed 4-5 days after weaning on this farm. The farmer's observations showed that the trial group with an addition of 1000 ppm Nor-Guard OV and 500 ppm ZnO performed just as well as the control group with 1000 ppm ZnO, concerning frequency and severity of diarrhea.

### Example 2 - Nor-Guard OV Powder

Trial on the farm "Bondeseje" in June, 2005. 1000 ppm Nor-Guard OV Powder was administered at weaning for the smallest piglets that often do not do well, which is expressed in a low survival rate or continuously low weight gain. The product was tested on 28 weak piglets. None of the weak piglets died which stands in contrast to the normal observations in these groups. According to the farmer's observations the small-trial pigs performed as well as the larger pigs.

### Example 3 - Nor-Guard OV 11 Powder

Trial on the farm "Svenstrup" from September 8 to September 29, 2005. Sixty-six weaned pigs were divided into two groups, the control group was fed with standard mixed feed and the trial group was fed with standard mixed feed and 15 ppm Nor-Guard OV 11 Powder, which was mixed in as a premix with wheat flour and sepiolite as carrying agents, and in a dosage of 2000 ppm of the premix.

| Results | | | |
|---|---|---|---|
| | Control group | Trial group | Diff.% |
| Number of pigs | 33 | 33 | ±0,0% |
| Av. start weight, kg | 6.88 | 6.92 | +0.6% |
| Av. end weight, kg | 10.72 | 11.38 | +6.2% |
| Growth / pig / day, grams | 159 | 213 | +34.0% |
| Feed consumption/pig/day, kg | 0.28 | 0.31 | +10.7% |
| Feed conversion, kg/kg growth | 1.75 | 1.46 | -16.6% |
| Mortality, number of pigs | 3 | 0 | - |

The results indicate an improved weight gain and feed conversion of 16.6% and 34.0%, respectively, when Nor-Guard OV 11 Powder was added to the feed. Moreover, the farmer confirms that the pigs fed with Nor-Guard OV 11 Powder thrived considerably better.

### Example 4 - Nor-Guard OV 12 Powder

Trial on the farm "Svenstrup Gods" from September 8 to September 29, 2005. 65 weaned pigs were divided into two groups, the control group was fed with standard mixed feed and the trial group was fed with standard mixed feed and 15 ppm Nor-Guard OV 12 Powder, which was mixed in as a premix with wheat flour and sepiolite as carrying agents, and in a dosage of 2000 ppm of the premix.

| Results | | | |
|---|---|---|---|
| | Control group | Trial group | Diff.% |
| Number of pigs | 32 | 33 | +3.1% |
| Av. start weight, kg | 6.88 | 6.88 | ±0.0% |
| Av. end weight, kg | 10.72 | 11.05 | +3.1% |
| Growth / pig / day, grams | 159 | 196 | +23.3% |
| Feed consumption/pig/day, kg | 0.28 | 0.32 | +14.3% |
| Feed conversion, kg/kg growth | 1.75 | 1.63 | -6.9% |
| Mortality, number of pigs | 3 | 0 | - |

The results indicate an improved weight gain and feed conversion of 23.3 % and 14.3 %, respectively, when Nor-Guard OV 12 Powder was added to the feed.

### Example 5 - Nor-Guard OV 21 Powder

Trial on the farm "Svenstrup Gods" from September 8 to September 29, 2005. 65 weaned pigs were divided into two groups, the control group was fed with standard mixed feed and the trial group was fed with standard mixed feed and 15 ppm Nor-Guard 21 Powder, which was mixed in as a premix with wheat flour and sepiolite as carrying agents, and in a dosage of 2000 ppm of the premix.

| Results | | | |
|---|---|---|---|
| | Control group | Trial group | Diff.% |
| Number of pigs | 33 | 33 | ±0.0% |
| Av. start weight, kg | 6.88 | 6.87 | -0.1% |
| Av. end weight, kg | 10.72 | 11.88 | +10.8% |
| Growth / pig / day, grams | 159 | 229 | +44.0% |
| Feed consumption/pig/day, kg | 0.28 | 0.32 | +14.3% |
| Feed conversion, kg/kg growth | 1.75 | 1.41 | -19.4% |
| Mortality, number of pigs | 3 | 2 | - |

The results indicate an improved weight gain and feed conversion of 44.0% and 19.4% respectively, when Nor-Guard OV 21 Powder was added to the feed.

### Example 6 - Nor-Guard OV 11 Powder

Trial on the farm "Gilleleje" from September 7 to September 28, 2005. 34 weaned pigs were divided into two groups, the control group was fed with standard mixed feed and the trial group was fed with standard mixed feed and 15 ppm Nor-Guard OV 11 Powder, which was mixed in as a premix with wheat flour and sepiolite as carrying agents, and in a dosage of 2000 ppm of the premix.

| Results | | | |
|---|---|---|---|
| | Control group | Trial group | Diff.% |
| *Number of pigs* | 17 | 17 | ±0.0% |
| Av. start weight, kg | 5.88 | 6.00 | +0.2% |
| Av. end weight, kg | 10.65 | 11.29 | +6.0% |
| Growth / pig / day, grams | 227 | 252 | +11.1% |
| Feed consumption/pig/day, kg | 0.32 | 0.36 | +11.1% |
| Feed conversion, kg/kg growth | 1.42 | 1.42 | ±0.0% |
| Mortality, number of pigs | 0 | 0 | ±0.0% |

The results indicate an improved weight gain of 11.1 % when Nor-Guard OV 11 Powder was added to the feed.

### Example 7 - Nor-Guard OV Powder

Trial on the farm "Gilleleje" from July 27 to August 17, 2005. 34 weaned pigs were divided into two groups, the control group was fed with the farm's standard mixed feed and the trial group was fed with standard mixed feed with 1000 ppm Nor-Guard OV Powder.

| Results | | | |
|---|---|---|---|
| | Control group | Trial group | Diff.% |
| *Number of pigs* | 17 | 17 | ±0.0% |
| Av. start weight, kg | 7.47 | 7.35 | -0.02% |
| Av. end weight, kg | 11.82 | 12.71 | +7.5% |
| Growth / pig / day, grams | 207.3 | 254.9 | +23.2% |
| Feed consumption/pig/day, kg | 0.34 | 0.40 | +15.9% |
| Feed conversion, kg/kg growth | 1.66 | 1.57 | -5.4% |
| Mortality, number of pigs | 0 | 0 | - |

The results indicate an improved weight gain and feed conversion of 23.2 % and 5.4 %, respectively, when Nor-Guard OV Powder was added to the feed. The feed intake in the two groups was the same during the first nine days of the trial. During the final twelve days of the trial the control group had a 20 % higher feed consumption than the trial group.

### Example 8 - Nor-Guard OV 11 Powder

Trial on the farm "Svenstrup Gods" from September 29 to October 10, 2005. 66 weaned pigs were divided into two groups, the control group was fed with standard mixed feed and the trial group was fed with standard mixed feed and 15 ppm Nor-Guard OV 11 Powder, which was mixed in as a premix with wheat flour and sepiolite as carrying agents, and in a dosage of 2000 ppm of the premix.

| Results | | | |
|---|---|---|---|
| | Control group | Trial group | Diff.% |
| *Number of pigs* | 33 | 33 | ±0.0% |
| Av. start weight, kg | 6.91 | 6.94 | +0.5% |
| Av. End weight, kg | 8.47 | 8.93 | +5.5% |
| Growth / pig / day, grams | 136 | 181 | +33.0% |
| Feed consumption/pig/day, kg | 0.22 | 0.21 | -4.7% |
| Feed conversion, kg/kg growth | 1.59 | 1.14 | -28.1% |
| Mortality, number of pigs | 3 | 0 | - |

The results indicate an improved weight gain and feed conversion of 33.0 % and 28.1 %, respectively, when Nor-Guard OV 11 Powder was added to the feed.

### Example 9 - Nor-Guard OV 11 Powder compared to single elements

Trial on the farm "Svenstrup Gods" from October 24 to October 31, 2005. One-hundred-and-eighty weaned pigs were divided into four groups as follows:
- control group fed with standard mixed feed,
- trial group O fed with standard mixed feed and 15 ppm Nor-Guard O Powder, which was mixed in the shape of a premix with wheat flour and sepiolite as carrying agents, and in a dosage of 2000 ppm of the premix.
- trial group V fed with standard mixed feed and 15 ppm Nor-Guard V Powder, which was mixed in the shape of a premix with wheat flour and sepiolite as carrying agents, and in a dosage of 2000 ppm of the premix.
- trial group OV fed with standard mixed feed and 15 ppm Nor-Guard OV 11 Powder, which was mixed in the shape of a premix with wheat flour and sepiolite as carrying agents, and in a dosage of 2000 ppm of the premix.

| Results: Control and trial group O | | | |
|---|---|---|---|
| | Control group | Trial group | Diff.% |
| Number of pigs | 45 | 45 | ±0.0% |
| Av. start weight, kg | 5.59 | 5.58 | -0.32% |
| Growth / pig / day, grams | 134.6 | 150.0 | +11.4% |
| Feed consumption/pig/day, kg | 0.23 | 0.25 | +8.7% |
| Feed conversion, kg/kg growth | 1.69 | 1.66 | -1.8% |
| Mortality, number of pigs | 1 | 1 | - |

The results indicate an improved weight gain, feed consumption and feed conversion of 11.4%, 8.7% and 1.8%, respectively, when Nor-Guard O Powder was added to the feed.

| Results: Control and trial group V | | | |
|---|---|---|---|
| | Control group | Trial group | Diff.% |
| Number of pigs | 45 | 45 | ±0.0% |
| Av. start weight, kg | 5.59 | 5.58 | -0.32% |
| Growth / pig / day, grams | 134.6 | 149.8 | +11.3% |
| Feed consumption/pig/day, kg | 0.23 | 0.25 | +8.7% |
| Feed conversion, kg/kg growth | 1.69 | 1.65 | -2.4% |
| Mortality, number of pigs | 1 | 0 | - |

The results indicate an improved weight gain, feed consumption and feed conversion of 11.3%, 8.7% and 2.4%, respectively, when Nor-Guard V Powder was added to the feed.

| Results: Control and trial group OV | | | |
|---|---|---|---|
| | Control group | Trial group | Diff.% |
| Number of pigs | 45 | 45 | ±0.0% |
| Av. start weight, kg | 5.59 | 5.58 | -0.32% |
| Growth / pig / day, grams | 134.6 | 173.3 | +28.8% |
| Feed consumption/pig/day, kg | 0.23 | 0.26 | +13.0% |
| Feed conversion, kg/kg growth | 1.69 | 1.48 | -12.4% |
| Mortality, number of pigs | 1 | 0 | - |

The results indicate an improved weight gain, feed consumption and feed conversion of 28.8%, 13.0% and 12.4%, respectively, when Nor-Guard OV11 Powder was added to the feed.

In summary, the results showed an improved weight gain, feed consumption and feed conversion when Nor-Guard OV11 Powder was added to the feed compared to inclusion of Nor-Guard O Powder or Nor-Guard V Powder or the control group.

## Claims

1. Use of a natural intestine-functional additive with antioxidative properties containing natural and/or nature identical active substances comprising;
- a first component comprising one or more of ground leaves of olives (*Olea europaea* L), an extract thereof obtainable by extraction with water, alcohol or a combination thereof , and a residual ground olive leaf product remaining after removal of one or more substances by said extraction, and;
- a second component comprising at least one of a primary pomace based on a by-product from wine production, which is a first residual product obtainable by pressing the juice out of the grapes, a secondary pomace, which is a second residual product obtainable by pressing of a residual product after fermentation of the primary pomace, and a tertiary pomace which is a third residual product obtainable by removal of one or more substances by extraction from the secondary pomace, said second component obtainable from grape plants (*Vitis vinifera* L),
as an additive for animal feed for improved feed conversion in the animals.

2. Use according to claim 1, wherein the first component is a combination of at least two of ground leaves of olives, an extract thereof obtainable by extraction with water, alcohol or a combination thereof, and a residual ground olive leaf product remaining after removal of one or more substances by said extraction.

3. Use according to claim 1 or 2, wherein the extract of ground olive leaves is obtainable by extraction in an alcohol/water mixture.

4. Use according to claim 3, wherein the alcohol/water mixture is in a 60:40 v/v ratio.

5. Use according to any of claims 1-4, wherein the second component is a combination of at least two of a primary pomace based on a by-product from wine production which is a first residual product obtainable by pressing the juice out of the grapes, a secondary pomace, which is a second residual product obtainable by pressing of a residual product after fermentation of the primary pomace, and a tertiary pomace which is a third residual product obtainable by removal of one or more substances by extraction from the secondary pomace.

6. Use according to any of the above claims, wherein the third pomace of the second component is obtainable as a residual product after conventional extraction process for the preparation of grape seed oil or natural colorants such as enocyanin extracted from the second pomace.

7. Use according to any of the above claims, wherein the quantity of the additive for 1000 kg feed contains 1-5000 g of the first component, calculated as the dry matter of a dried extract.

8. Use according to any of the above claims, wherein the quantity of the additive for 1000 kg feed contains 1-5000 g of the second component, calculated as the dry matter of a dried extract.

## Patentansprüche

1. Verwendung eines auf den Darm einwirkenden natürlichen Zusatzstoffs mit antioxidativen Eigenschaften, der natürliche und/oder naturidentische aktive Substanzen enthält, mit:
- einem ersten Bestandteil mit einem oder mehreren zermahlenen Olivenblättern (*Olea europaea* L), einem Extrakt davon, erhältlich durch Extraktion mit Wasser, Alkohol oder einer Kombination davon, und einem Rückstand von zermahlenen Olivenblättern, der nach Entfernung einer oder mehrerer Substanzen durch besagte Extraktion zurückbleibt, sowie:
- einem zweiten Bestandteil mit mindestens einem der folgenden: einem primären Trester auf der Grundlage von einem Nebenprodukt aus der Weinbereitung, der ein erster Rückstand ist, erhältlich durch Pressen des Saftes aus den Trauben, einem sekundären Trester, der ein zweiter Rückstand ist, erhältlich durch Pressen eines Rückstandes nach der Gärung des primären Tresters, und einem tertiären Trester, der ein dritter Rückstand ist, erhältlich durch Entfernung einer oder mehrerer Substanzen durch Extraktion aus dem sekundären Trester, wobei der zweite Bestandteil aus Weinreben (*Vitis vinifera* L) erhältlich ist,
als Zusatzstoff für Tierfutter zur besseren Futterverwertung bei den Tieren.

2. Verwendung gemäß Anspruch 1, wobei der erste Bestandteil eine Kombination von mindestens zwei der folgenden ist: zermahlene Olivenblätter, ein Extrakt davon, erhältlich durch Extraktion mit Wasser, Alkohol oder einer Kombination davon, und ein Rückstand von zermahlenen Olivenblättern, der nach Entfernung einer oder mehrerer Substanzen durch besagte Extraktion zurückbleibt.

3. Verwendung gemäß Anspruch 1 oder 2, wobei der Extrakt von zermahlenen Oliven blättern durch Extraktion in einer Alkohol-/Wassermischung erhalten wird.

4. Verwendung gemäß Anspruch 3, wobei die Alkohol-/Wassermischung in einem 60:40-Verhältnis pro Volumen ist.

5. Verwendung nach einem beliebigen der Ansprüche 1-4, wobei der zweite Bestandteil eine Kombination von mindestens zwei der folgenden ist: einem primären Trester auf der Grundlage von einem Nebenprodukt aus der Weinbereitung, der ein erster Rückstand ist, erhältlich durch Pressen des Saftes aus den Trauben, einem sekundären Trester, der ein zweiter Rückstand ist, erhältlich durch Pressen eines Rückstandes nach der Gärung des primären Tresters, und einem tertiären Trester, der ein dritter Rückstand ist, erhältlich durch Entfernung einer oder mehrerer Substanzen durch Extraktion aus dem sekundären Trester.

6. Verwendung nach einem beliebigen der obigen Patentansprüche, wobei der dritte Trester des zweiten Bestandteils als Rückstand nach einem konventionellen Extraktionsverfahren zur Herstellung von Traubenkernöl erhältlich ist oder natürlichen Farbstoffen wie zum Beispiel Enocyanin, das aus dem zweiten Trester extrahiert wird.

7. Verwendung nach einem beliebigen der obigen Patentansprüche, wobei die Menge des Zusatzstoffs für 1000 kg Futter 1-5000 g des ersten Bestandteils enthält, berechnet als der Trockenstoff eines getrockneten Extrakts.

8. Verwendung nach einem beliebigen der obigen Patentansprüche, wobei die Menge des Zusatzstoffs für 1000 kg Futter 1-5000 g des zweiten Bestandteils enthält, berechnet als der Trockenstoff eines getrockneten Extrakts.

## Revendications

1. Utilisation d'un additif naturel qui agit sur l'intestin avec des propriétés antioxydantes contenant des substances actives naturelles et/ou identiques aux substances naturelles, comprenant :
- un premier composant comprenant un ou plusieurs parmi les suivants : feuilles d'olivier moulues (*Olea europaea* L), un extrait de cela obtenu par extraction avec de l'eau, de l'alcool ou une combinaison de ces derniers, et un produit de feuille d'olivier moulue résiduel qui reste après l'enlèvement de l'une ou de plusieurs substances par ladite extraction, et
- un deuxième composant comprenant au moins un parmi les suivants : marc primaire basé sur un sous-produit de la production de vin, qui est un premier résidu, obtenu par pressage du jus des grains de raisin, un marc secondaire, qui est un deuxième résidu, obtenu par pressage d'un résidu après fermentation du marc primaire, et un marc tertiaire qui est un troisième résidu, obtenu par l'enlèvement d'une ou de plusieurs substances par l'extraction du marc secondaire, ledit deuxième composant étant obtenu à partir de vignes (*Vitis vinifera* L),
comme additif destiné à l'alimentation pour animaux, pour l'amélioration de la conversion alimentaire chez les animaux.

2. Utilisation selon la revendication 1, où le premier composant est une combinaison d'au moins deux parmi les suivants : feuilles d'olivier moulues, un extrait de cela obtenu par extraction avec de l'eau, de l'alcool ou une combinaison de ces derniers, et un résidu de feuilles d'olivier moulues qui reste après l'enlèvement de l'une ou de plusieurs substances par ladite extraction.

3. Utilisation selon la revendication 1 ou 2, où l'extrait de feuilles d'olivier moulues peut être obtenu par extraction dans un mélange alcool/eau.

4. Utilisation selon la revendication 3, où le mélange alcool/eau a un rapport 60:40 v/v.

5. Utilisation selon l'une quelconque des revendications 1 à 4, où le deuxième composant est une combinaison d'au moins deux parmi les suivants : marc primaire basé sur un sous-produit de la production de vin qui est un premier résidu, obtenu par pressage du jus des grains de raisin, un marc secondaire qui est un deuxième résidu, obtenu par pressage d'un résidu après fermentation du marc primaire, et un marc tertiaire qui est un troisième résidu, obtenu par l'enlèvement de l'une ou de plusieurs substances par extraction du marc secondaire.

6. Utilisation selon l'une quelconque des susdites revendications, où le troisième marc du deuxième composant peut être obtenu comme un produit résiduel après un procédé d'extraction conventionnel pour la préparation d'huile de pépins de raisin ou de colorants naturels comme l'énocyanine extrait du deuxième marc.

7. Utilisation selon l'une quelconque des revendications susmentionnées, où la quantité de l'additif pour 1000 kg d'aliments contient 1-5000 g du premier composant, calculée comme la matière sèche d'un extrait séché.

8. Utilisation selon l'une quelconque des revendications susmentionnées, où la quantité de l'additif pour 1000 kg d'aliments contient 1-5000 g du deuxième composant, calculée comme la matière sèche d'un extrait séché.
